# EUROPEAN PATENT APPLICATION

(11) **EP 1 857 550 A1**
(43) Date of publication of application: **21.11.2007**
(21) Application number: 06714059.0
(22) Date of filing: 14.02.2006
(51) Int. Cl.: C12N 15/12, C12N 15/29, C12N 15/53, C12N 15/62, C12N 15/82, A01H 5/00

(54) **TRANSGENIC PLANT CAPABLE OF DETECTING ENVIRONMENTAL BURDEN CHEMICAL**

(30) Priority: 14.02.2005 JP 2005036338
(71) Applicant: SUNTORY LIMITED, Osaka-shi, Osaka 530-8203 (JP)
(72) Inventor: TOGAMI, Junichi, Takatsuki-shi, Osaka 5690814 (JP); OKUHARA, Hiroaki, Nagaoka-shi, Niigata 9400834 (JP); TANAKA, Yoshikazu, Otsu-shi, Shiga 5200246 (JP)
(74) Representative: Vossius & Partner
(86) International application number: PCT/JP2006/302918
(87) International publication number: WO 2006/085699

(57) **Abstract**

The present invention provides a plant transformation vector for producing a plant for detecting an environmental chemical by a change of flower color, the plant transformation vector comprising an AhR gene expressibly incorporated therein, an AhR ligand stimulus-inducible promoter, and an expression suppressive factor for suppressing the expression of a gene involved in the synthesis of a desired flower pigment, the factor being expressibly incorporated therein by the promoter.

## Description

### TECHNICAL FIELD

The present invention relates to a plant transformation vector for producing a plant for detecting an environmental chemical by a change of flower color, a transgenic plant transformed using such a vector, a method for detecting an environmental chemical using such a transgenic plant, and the like. More specifically, the present invention relates to a technology for easily detecting any of dioxins/dioxin-like compounds and polycyclic aromatic hydrocarbons, which are AhR ligands present in the environment, by using a plant having a group of genes including a gene of an aryl hydrocarbon receptor (AhR) introduced thereto.

### BACKGROUND ART

In the process of industrial development until today, various chemical substances were released to the environment. Among them are many chemical substances which remain and are accumulated in the environment and threaten the health of biological organisms including the human being (hereinafter, referred to as "environmental chemicals"). Especially, dioxins/dioxin-like compounds and polycyclic aromatic hydrocarbons have extremely adverse effects on the environment at a very low concentration and have been socially considered as a problem. Among the environmental chemicals, dioxins/dioxin-like compounds and polycyclic aromatic hydrocarbons (including benzpyrene, methylcholanthrene, and the like) exhibit various types of *in vivo* toxicity such as immunotoxicity, teratogenicity, carcinogenicity and the like on mammals, and are considered to have adverse influence on the ecosystem as well as the human health.

Acute toxicity can be compared based on the LD (lethal dose) of compounds. Among dioxins/dioxin-like compounds, 2,3,7,8-TCDD is considered to have an especially high level of toxicity, and reaches LD₅₀ (50% lethal dose) at a very low concentration, although the concentration varies depending on the type of biological organism. For example, for monkeys similar to humans, 2,3,7,8-TCDD reaches LD₅₀ at a concentration of 50 to 70 µg/kg, which is very low as compared with other toxic substances. Dioxins, which exhibit various types of toxicity including carcinogenicity, teratogenicity, hormone-like effects and the like, are considered to influence health when accumulated *in vivo* even at a concentration which is too low to exhibit acute toxicity. Other dioxins and dioxin-like compounds including coplanar PCB are known to exhibit various types of toxicity similar to those of 2,3,7,8-TCDD, although the level of toxicity is varied ("Environmental Hormone and Dioxins" Kagaku-dojin Publishing Company, Inc., 1999).

Under such circumstances, technologies for monitoring the distribution and kinetics of dioxins/dioxin-like compounds and polycyclic aromatic hydrocarbons present in the environment at a low concentration have been desired.

Conventionally, dioxins/dioxin-like compounds and polycyclic aromatic hydrocarbons are monitored by collecting samples from many points in the target area to be monitored, transferring the samples to the experimental facilities, and then performing detection and quantification operations by analysis using instruments. Such analysis using instruments, although being high in sensitivity and precision, requires analysis equipment and skill, is time-consuming, and is costly due to the instruments and reagents. Currently, one request for analysis costs several hundred thousand yen. For these reasons, a simple, quick, highly-sensitive, and low-cost method is desired.

Biological organisms have a function of recognizing and metabolizing dioxins/dioxin-like compounds and polycyclic aromatic hydrocarbons. A method of using such a function to monitor dioxins/dioxin-like compounds and the like has little risk of secondary contamination which would be caused when a great amount of organic solvents is used, and therefore does not impose much load on the environment and is likely to be approved by the public. So far, methods of using such a function of biological organisms to monitor the environmental chemicals have been developed. For example, for an assay of endocrine disruptive substances, which is one type of environmental chemical, methods which use, as an index, the responsiveness to receptors involved in the endocrine system functions such as female hormone (estrogen), male hormone (androgen), thyroid gland hormone and the like are known. Immunochemical measurement methods, specific to environmental chemicals, which utilize the specificity of *in vivo* antigen-antibody reaction, are also known. For dioxins/dioxin-like compounds, test kits for *in vitro* assays using receptors, for example, Ecoassay Dioxin ELISA Kit (Otsuka Pharmaceutical Co., Ltd.), Ah Immunoassay Kit (Wako Pure Chemical Industries, Ltd.), Dioxin/Furan EIA kit, 60 test, Df1-60 (Kanto Chemical Co., Inc.) and the like are commercially available.

However, the above-mentioned assays perform the measurement using reagents and instruments suitable for each type of analysis after the collected samples are taken back to the laboratory from the collection site. These assays cannot be used in a location where there are no experimental facilities or the use thereof is substantially impossible (for example, an outdoor site far from the experimental facilities). In addition, according to these assays, the measurement results are only found after the analysis is performed in the experimental facilities, and a quick on-site measurement at the collection site is impossible.

As described above, dioxins/dioxin-like compounds exhibit strong toxicity even at a low concentration, especially on mammals. When exhibiting toxicity, dioxins/dioxin-like compounds specifically bind to a receptor AhR in the cell. Then, an AhR-dioxin complex including a dioxin or a dioxin-like compound as a ligand forms a hetero dimer together with an AhR nuclear translocator (Amt) protein, which is translocated into the nucleus. The hetero dimer binds a specific DNA sequence via a DNA-binding domain in the AhR, and activates transcription of a structural gene which is present on the 3' side of the specific DNA sequence via a transcriptional activation domain in the AhR (J. Biol. Chem., 270, 29270-29278, 1995).

Attempts for detecting dioxins/dioxin-like compounds utilizing the feature that the AhR of the mammals can recognize dioxins/dioxin-like compounds of a very low concentration as ligands have been reported and partially succeeded (Japanese Laid-Open Patent Publication No. 2004-89068). The AhR ligand-specific gene inductive expression system described in Japanese Laid-Open Patent Publication No. 2004-89068 operates as follows. The structure of the AhR is divided into three functional domains of (i) DNA-binding domain, (ii) ligand-binding control domain, and (iii) transcriptional activation domain, from the amino acid terminus thereof. Among these domains, the ligand-binding control domain is used as a basic functional domain, and appropriate functional domains such as the DNA-binding domain, the transcriptional activation domain of herpes virus VP16 factor and the like are merged thereto. This publication describes that in the case of tobacco including a vector, to which E. coli β-glucuronidase (GUS) gene is induced in the presence of dioxin or a dioxin-like compound, the GUS gene was expressed with 5 nM 20-methylcholanthrene (used as an analog of dioxin or a dioxin-like compound).

However, this method requires sampling and GUS-dyeing of the leaves of the plant to be examined in order to check whether or not dioxin or a dioxin-like compound is present, and so is not simple.

The color of flowers is mainly derived from three types of compounds, i.e., anthocyanin, carotenoid and betalain. Among these compounds, anthocyanin has the widest hue and contributes to flower colors of many species of plants. The biosynthesis route of flavonoid, which contains anthocyanin, has been studied in detail, and the route is stored in higher plants. In addition, enzyme genes involved in the synthesis of anthocyanin have been cloned. It is known that the structure and amount of flavonoid accumulated in the flower can be altered to change the flower color by artificially adjusting the expression of such genes (Plant Cell Physiol. 39, 1119-1126, 1998). Using such a technique, blue carnation and orange petunia which do not naturally exist are created. However, there is no report that such a color change was used for detecting dioxin or a dioxin-like compound or has interaction with dioxin or a dioxin-like compound.

It has been reported that the flower colors were whitened or paled by suppressing the expression of genes of chalcone synthase, flavanone 3-hydroxylase (F3H), dihydroflavonol reductase, and anthocyanidin synthase, among the structural genes involved in the synthesis of anthocyanin (Plant Cell Physiol. 39, 1119-1126, 1998; Plant Cell Physiol. 44, s122, 2003; Plant Biotechnology, 21, 377-387, 2004). It has also been reported that the flower colors were changed by suppressing the expression of genes involved in the synthesis of anthocyanin using an RNAi method (Plant Cell Physiol. 44, s122, 2003). However, these documents do not describe or suggest that such a change of flower colors could be used for detecting dioxin or a dioxin-like compound or the like.

It has been indicated by grafting tests and the like that a silencing signal of extrinsic genes, such as GUS or the like, in the lower part of a plant including rhizosphere is translocated to the upper part of the plant through the inside thereof and causes silencing also in the upper part of the plant (EMBO J. 16, 4738-45, 1997). However, there is no report that the expression of extrinsic flavonoid metabolic genes introduced from outside is suppressed by the RNAi method in rhizosphere and the signal is translocated to the part above the ground to change the flower color.

It is considered that by combining the above-described dioxin transcription system of inducing dioxin or a dioxin-like compound with cytochrome P450 gene such as flavonoid 3'-5'-hydroxylase or the like, petunia or the like, which changes the flower color from pink to blue in the presence of dioxin or a dioxin-like compound, is created (Bio-oriented Technology Research Advancement Institution [online] [searched on January 27, 2005], Internet <URL: http://brain.naro.affrc.go.jp/Tokyo/gijutu/14kadai/hp3/kobetu/10-12.htm#kobetsu11>). However, creation of such plants has not been realized yet.

### DISCLOSURE OF THE INVENTION

Under the above-described circumstances, it is desired to develop a simple, quick, highly-sensitive and low-cost method for detecting any of dioxins/dioxin-like compounds and polycyclic aromatic hydrocarbons in the environment, while allowing an on-site measurement and not imposing much load on the environment.

The present invention, made in order to overcome the above-described problems, provides a plant transformation vector as described below, a transgenic plant transformed with such a vector, and a method for detecting an environmental chemical using such a transgenic plant.
(1) A plant transformation vector for producing a plant for detecting an environmental chemical by a change of flower color, the plant transformation vector comprising an AhR gene expressibly incorporated therein, an AhR ligand stimulus-inducible promoter, and an expression suppressive factor for suppressing the expression of a gene involved in the synthesis of a desired flower pigment, the factor being expressibly incorporated therein by the promoter.
(2) The plant transformation vector according to (1) above, comprising genes coding for a DNA-binding domain, an AhR ligand-binding domain and a transcriptional activation domain, also comprising a nucleic acid coding for a double strand RNA (dsRNA) having an RNA interference effect on the gene involved in the synthesis of the flower pigment; wherein the nucleic acid is under the control of the AhR ligand stimulus-inducible promoter, and the DNA-binding domain binds the AhR ligand stimulus-inducible promoter in response to an AhR ligand.
(3) The plant transformation vector according to (1) above, wherein the gene involved in the synthesis of the flower pigment is a gene involved in the biosynthesis of anthocyanin.
(4) The plant transformation vector according to (3) above, wherein the gene involved in the biosynthesis of anthocyanin is a gene coding for flavanone 3-hydroxylase (F3H) or flavonoid 3',5'-hydroxylase (F3',5'H).
(5) The plant transformation vector according to (1) above, wherein an AhR ligand is any of dioxins/dioxin-like compounds, and/or polycyclic aromatic hydrocarbons, or an analog thereof.
(6) The plant transformation vector according to (1) above, comprising XD₄V or XD₅V
(7) The plant transformation vector according to (1) above, wherein the AhR ligand stimulus-inducible promoter includes a LexA promoter sequence.
(8) The plant transformation vector according to (1) above, further comprising at least one desired gene.
(9) The plant transformation vector according to (8) above, wherein the desired gene is a drug resistance gene.
(10) The plant transformation vector according to (8) above, wherein the desired gene is carnation THC2'GT gene.
(11) The plant transformation vector according to (1) above, wherein the plant is petunia, torenia or verbena.
(12) A transgenic plant for changing the flower color thereof in response to an AhR ligand, the transgenic plant being transformed with the vector according to (1) above.
(13) The transgenic plant according to (12) above, which is petunia, torenia or verbena.
(14) A reproduction material of the transgenic plant according to (12) above.
(15) A method for detecting an AhR ligand in soil using a transgenic plant, the method comprising the step of transplanting the transgenic plant according to (12) above to soil to be tested, cultivating the transgenic plant, and observing a change of flower color.
(16) The method according to (15) above, wherein the AhR ligand is any of dioxins/dioxin-like compounds, and/or polycyclic aromatic hydrocarbons, or an analog thereof.

A method for changing a flower color of a plant using an expression suppressive function on a gene involved in a flower pigment, which is induced in response to dioxins/dioxin-like compounds and polycyclic aromatic hydrocarbons, is provided by the present invention for the first time in history. A method for detecting an environmental chemical according to the present invention provides effects of allowing an on-site measurement and not imposing much load on the environment. The detection method according to the present invention also provides effects of being simple, quick, highly-sensitive and low-cost.

### BRIEF DESCRIPTION OF THE DRAWINGS

Fig. 1 schematically shows structures of expression constructs used in Reference Examples 1 and 2 (PAB, PD₄B, PD₅B and TAG) and expression constructs used in Examples 1, 2 and 3 (PD₄F, PD₅F, TD₄F, TD₅F, PD₅dB and SkD₅dB).
Fig. 2 schematically shows an RNA interference action in the present invention.
Fig. 3 shows photos showing the flower color obtained when petunia and torenia transformed with a vector according to the present invention were cultivated in the absence and in the presence of dioxin or a dioxin-like compound. (a) and (b) correspond to Examples 1 and 2, and (c) corresponds to Example 3.

In the figures, alphabetical codes (: size [accession number]) represent the following.
a: AhR: 1.2kb, D4: 1.2kb, D5: 1.5kb [D38417]
b: Arnt: 2.1kb [U14333]
c: XRE: 0.1kb
d: 5'-UTR (U)
e: CaMV35S-promoter (35S-P): 0.2kb [E05206]
f: mas-terminator (T2): 0.8kb
g: Mac-promoter (Mac-P): 1.2kb
h: NPTII: 1.0kb [U09635]
i: HPT: 1.0kb [AF309825]
j: LexA (X): 0.6kb [AF309825]
k: LexA binding domain (8xLexA): 0.3kb [AF309825]
1: VP16 (V): 0.2kb [AF309825]
m: GFP: 0.7kb [AY598428]
n: butterfly pea F3'S'H: 1.7kb
o: Torenia dsANS: 1.0kb
p: Torenia dsF3H(Tr):1.7kb
q: Petunia dsF3H(Pt): 1.6kb
r: Verbena dsF3H(Ha): 1.9kb
s: Carnation THC2'GT: 1.7kb
t: Nos-promoter used as the promoter of NPTII and HPT: 0.3kb [U09365]
u: Nos-terminater (T1) used as the promoter ofNPTII and HPT: 0.3kb [U09365]
v: G10-90 promoter (G10-90-P): 0.2kb [AF309825]
w: Ω sequence (inserted between G10-90-P and XD₄V/XD₅V)
x: nuclear translocator signal (inserted between X and D₄/D₅ of XD₄V/XD₅V)
y: RbcS E9-terminator (T3): 0.5kb [AF309825]
z: RbcS 3A-terminator (T4): 0.5kb [AF309825]
α: 3' non-translated region of rat-derived glucocorticoid receptor [AF309825]
β: butterfly pea dsF3'5'H: 1.5kb

### BEST MODE FOR CARRYING OUT THE INVENTION

As described above, there is a report that by combining the above-described dioxin or a dioxin-like compound-inducible transcription system with cytochrome P450 gene such as flavonoid 3'-5'-hydroxylase or the like, petunia or the like, which changes the flower color from pink to blue in the presence of dioxin or a dioxin-like compound, is created. However, in the experiments actually performed by the present inventors, such a change of flower color was not observed using this technique because the induction level of the flavonoid 3'-5'-hydroxylase gene by dioxin or a dioxin-like compound was low (see Reference Examples 1 and 2 in this specification).

The present inventors accumulated experiments based on the concept that using a promoter that induces an expression of a gene in response to a stimulus of an environmental chemical, an expression of a gene involved in the synthesis of flower pigment of a subject plant can be suppressed by the effect of the promoter such that the flower color is changed and the environmental chemical can be detected. The present inventors succeeded in changing the flower color by inducing the suppression of the expression of the gene relating to the endogenous flower pigment using RNA interference (RNAi), which is caused in response to an AhR ligand (for example, dioxin or a dioxin-like compound, and/or polycyclic aromatic hydrocarbon), which is an environmental chemical; and thus completed the present invention.

Namely, the present invention provides a plant transformation vector for producing a plant for detecting an environmental chemical by a change of flower color, the plant transformation vector comprising an expressibly incorporated AhR gene, an AhR ligand stimulus-inducible promoter, and an expression suppressive factor for suppressing the expression of a gene involved in the synthesis of a desired flower pigment, which have been expressibly incorporated therein by the promoter.

Herein, the term "environmental chemical" refers to a substance which imposes load on the ecosystem, for example, threatens the health of biological organisms including the human being, while remaining and being accumulated in the environment after being released thereto. Representative environmental chemicals include dioxins/dioxin-like compounds and polycyclic aromatic hydrocarbons, which are AhR ligands.

A preferable "AhR gene" which can be used for a plant transformation vector according to the present invention will be described. Here, it is assumed that the structure of the AhR is divided into three domains of (1) DNA-binding domain, (2) AhR ligand-binding domain, and (3) transcriptional activation domain. A preferable AhR gene may be a recombinant AhR gene coding for an AhR fused protein which includes (a) an appropriate AhR ligand-binding domain derived from any of various species including human, mouse, rat, guinea pig and the like which can be used as a basic structural element, and (b) homologous or heterologous functional region fused thereto, such as an appropriate DNA-binding domain (e.g., DNA-binding domain of mouse AhR or DNA-binding domain of E. coli LexA protein) and an appropriate transcriptional activation domain (e.g., transcriptional activation region of herpes virus VP16 factor). Needless to say, the recombinant AhR gene does not need to encode a fused protein, and the above-mentioned three domains may be homologous.

Herein, the term "AhR ligand stimulus-inducible promoter" means an inductive promoter responsive to an inductive factor such as an AhR ligand to cause transcription of a transcription unit that is under the control of the promoter in accordance with a predetermined molecular biological process.

Herein, the term "expressibly incorporated" gene refers to a gene incorporated into a vector such that the gene can be expressed in a cell to which the gene is introduced. The "gene expressibly incorporated (into the vector)" can be realized by, for example, locating an expression controlling factor, such as an appropriate promoter for controlling the expression of the gene, at a position at which the expression of the gene in the vector is controllable (e.g., upstream with respect to the gene).

Herein, the term "gene involved in the synthesis of a flower pigment" refers to any of a group of genes involved in the biosynthesis of a pigment for causing the flower to develop a color. Such genes include, for example, genes for enzymes involved in the biosynthesis of a pigment included in a group of pigments such as anthocyanin, carotenoid and betalain. Preferable examples of the gene involved in the synthesis of a flower pigment which can be used in the present invention include genes involved in the biosynthesis of anthocyanin. The reason is that anthocyanin has the widest hue among the compounds included in the above-mentioned group of pigments and contributes to flower colors of many species of plants. The biosynthesis route of flavonoid, which contains anthocyanin, has been studied in detail, and the route is stored in higher plants. In addition, genes for enzymes involved in the synthesis of anthocyanin have been cloned. The expression of these genes can be artificially controlled (see, for example, Plant Cell Physiol. 39, 1119-1126, 1998). Genes known as being involved in the biosynthesis of anthocyanin include chalcone synthase, flavanone 3-hydroxylase (F3H), dihydroflavonol reductase, anthocyanidin synthase, flavonoid 3'-5'-hydroxylase (F3',5'H) and the like. Preferably, F3H or F3',5'H gene is used. F3H is an enzyme for catalyzing the reaction of converting naringenin into dihydrokaempferol. By suppressing the expression of F3H gene, the biosynthesis of flavonoid, which is downstream of dihydrokaempferol biosynthesis, is inhibited. Thus, the accumulation of anthocyanin is inhibited. As a result, the flower color exhibited by the accumulation of anthocyanin, which is a colored pigment, is paled and becomes closer to white. F3',5'H is an enzyme for catalyzing the reaction of converting dihydrokaempferol into dihydromyricetin. By promoting the expression of F3',5'H gene, the flower color of the species of plants having a low efficiency of pigment conversion from dihydrokaempferol can be changed from a pale color to dark purple. By introducing a construct for suppressing the expression of F3',5'H gene into the system in which the flower color has been once changed to dark purple by promoting the expression, the flower color can be changed from dark purple back to the pale color.

Herein, the term "expression suppressive factor" refers to a DNA sequence to be inserted into a vector according to the present invention in order to suppress the expression of a specific gene. Examples of the expression suppressive factor include a DNA sequence coding for dsRNA for inducing an RNA interference effect on a specific gene in the RNAi method, a DNA sequence coding for antisense RNA which operates to cause an expression suppressive action on a specific gene in an antisense method, a DNA sequence having the same sequence as that of a specific gene in a co-suppression method, and the like. In general, currently known methods for suppressing the expression of an endogenous gene in a plant include the antisense method, the co-suppression method, the RNAi method and the like. Especially, the RNAi method is known as suppressing the expression of endogenous genes most efficiently (Plant Cell Physiol. 44, s122, 2003). Thus, as the "expression suppressive factor" according to the present invention, the DNA sequence coding for dsRNA for inducing an RNA interference effect is preferable. The gene, which is to be suppressed from being expressed, may be an extrinsic gene introduced from outside and expressed, as well as an endogenous gene. According to preferable techniques for producing a transgenic plant in the case of an extrinsic gene, the extrinsic gene and the gene coding for dsRNA are ligated to the same vector and introduced into the plant, or the extrinsic gene and the gene coding for dsRNA are introduced into the vector in two stages at different times. The method is not limited to these.

Now, the present invention will be described in more detail.

### (Construction of vector)

According to the present invention, an environmental chemical-inducible gene expression system is constructed. For this gene expression system, the transcription system described in Japanese Laid-Open Patent Publication No. 2004-89068 is preferably used although the transcription system is not specifically limited to this. For example, according to the environmental chemical inducible gene expression system of the present invention, a vector comprising a series of sequences is constructed by linking (1) a gene coding for a fused protein, which comprises a DNA-binding region, a nuclear localization signal sequence, an AhR ligand-binding control region, and a transcriptional activation region, being ligated downstream to a first promoter sequence for promoting constitutive expression and (2) an expression suppressive factor for, when introduced into the plant, suppressing the expression of a gene involved in the synthesis of a flower pigment of a plant, being ligated downstream to a second promoter sequence including a sequence specifically binding to the DNA-binding region. For constructing such a vector, the description in Japanese Laid-Open Patent Publication No. 2004-89068 can be referred to.

A vector according to a preferable embodiment of the present invention is constructed such that (1) the vector includes a gene coding for a DNA-binding domain, an AhR ligand-binding domain, and a transcriptional activation domain; (2) the vector includes a nucleic acid coding for dsRNA capable of inducing an RNA interference effect on a gene involved in the synthesis of a flower pigment; and (3) the nucleic acid is under the control of the AhR ligand stimulus-inducible promoter, and the DNA-binding domain binds the inductive type promoter in response to the AhR ligand.

Herein, the term "AhR ligand" refers to a substance which binds an AhR to induce the bioactivity of the AhR. Preferable examples of the "AhR ligand" according to the present invention include dioxins/dioxin-like compounds and polycyclic aromatic hydrocarbons. Representative examples of dioxins/dioxin-like compounds include polychlorinated dibenzo-para-dioxins (PCDDs), polychlorinated dibenzofrans (PCDFs), co-planar PCBs (Co-PCBs), and the like. Representative examples of polycyclic aromatic hydrocarbons include benzpyrene, methylcholanthrene, and the like. Herein, an analog of the above-mentioned compounds is also referred to as an "AhR ligand" as long as such an analog binds an AhR and induces the bioactivity of the AhR. Examples of such a compound include 20-methylcholanthrene (MC), β- naphtoflavone, indigo and the like.

Herein, the term "AhR ligand-binding domain" means a ligand-binding domain of an AhR derived from any of various species including human, mouse, rat, guinea pig and the like. An AhR ligand-binding domain of any species can be used as long as the domain is of AhR protein capable of activating an AhR ligand stimulus-inducible promoter. An AhR ligand is specifically bound to the AhR ligand-binding domain to form an AhR-AhR ligand complex. The "AhR ligand-binding domain" according to the present invention encompasses a variant formed of an amino acid sequence obtained as a result of introducing deletions, additions or substitutions of one or more amino acids (for example, 1 to several amino acids (e.g., 6 amino acids)) in the amino acid sequence of the above-mentioned AhR ligand-binding domain, in which the deletions, additions or substitutions are introduced to the degree at which the inherent functions of the AhR ligand are not lost.

Herein, the term "gene coding for an AhR ligand-binding domain" means a gene coding for the above-described AhR ligand-binding domain. In the present invention, for example, a DNA sequence coding for an amino acid sequence of position 83 to position 593 of an amino acid sequence of mouse AhR (PDB Accession No. D38417) and an amino acid sequence of position 83 to position 493 of the amino acid sequence of mouse AhR can be used.

Herein, the term "DNA-binding domain" refers to a domain corresponding to a region in an amino acid sequence of AhR protein, which binds to a predetermined sequence of an inductive promoter. In the present invention, a DNA-binding domain derived from any of various species including human, mouse, rat, guinea pig and the like can be used. When an AhR-AhR ligand complex is formed, a DNA-binding domain binds an inductive promoter. The "DNA-binding domain" according to the present invention encompasses a variant formed of an amino acid sequence obtained as a result of deletion, addition or substitution of one or more amino acids (for example, 1 to several amino acids (e.g., 6 amino acids)) with respect to the amino acid sequence of the above-mentioned DNA-binding domain to the degree at which the inherent functions of the DNA-binding domain are not lost.

Herein, the term "gene coding for a DNA-binding domain" means a DNA sequence coding for the DNA-binding domain. A gene coding for a DNA-binding domain is appropriately selected depending on the inductive promoter used. For example, a DNA sequence coding for a DNA-binding domain of mouse AhR (an amino acid sequence of amino acid residues No. 1 to No. 82) or a DNA sequence coding for a DNA-binding domain of LexA (an amino acid sequence of amino acid residues No. 1 to No. 202 of bacteria repressor LexA) is appropriately used.

Herein, the term "transcriptional activation domain" refers to a domain in an AhR which is considered to activate an inductive promoter when an AhR-AhR ligand complex binds the inductive promoter in the DNA-binding domain. In the present invention, a transcriptional activation domain derived from any of various species including human, mouse, rat, guinea pig and the like can be used. The "transcriptional activation domain" according to the present invention encompasses a variant formed of an amino acid sequence obtained as a result of introducing deletions, additions or substitutions of one or more amino acids (for example, 1 to several amino acids (e.g., 6 amino acids)) in the amino acid sequence of the above-mentioned transcriptional activation domain, in which the deletions, additions or substitutions are introduced to the degree at which the inherent functions of the transcriptional activation domain are not lost.

Herein, the term "gene coding for a transcriptional activation domain" refers to a gene coding for the above-mentioned transcriptional activation domain. In the present invention, a DNA-sequence coding for a transcriptional activation domain derived from any of various species can be used. Preferably, a DNA sequence corresponding to a DNA sequence coding for a transcriptional activation domain of herpes simplex virus VP16 protein can be used; and more preferably, a DNA sequence corresponding to a DNA sequence coding for amino acids No. 413 to No. 490 in the transcriptional activation domain of herpes simplex virus VP16 protein can be used.

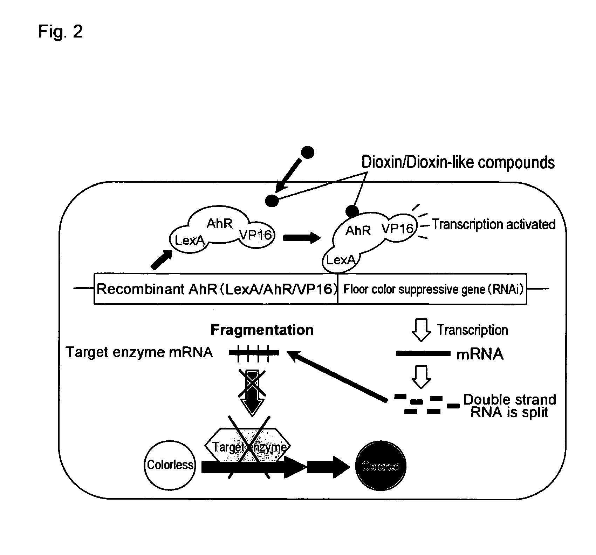

Herein, the term "RNA interference" is used in the usual meaning in the field, i.e., refers to a phenomenon that a double strand RNA (dsRNA) promotes specific decomposition of a complementary target mRNA to specifically suppress the expression of target protein. In the present invention, an inductive promoter is activated by an inductive factor to transcribe dsRNA having an RNA interference effect on a gene involved in the synthesis of a flower pigment (e.g., a gene involved in the biosynthesis of flavanone 3-hydroxylase) and thus to inhibit the expression of a gene involved in the synthesis of an endogenous flower pigment (see Fig. 2).

According to the present invention, the inductive promoter, when specifically binding to the DNA-binding domain, is influenced by the effect of a transcriptional activation domain and activates the transcription of a structural gene under the control thereof. In the present invention, an appropriate inductive promoter is selected depending on a sequence used as a DNA sequence coding for the DNA-binding domain. For example, when a DNA sequence coding for a DNA-binding domain of mouse AhR (amino acids No. 1 to No. 82) is used as the DNA sequence coding for the DNA-binding domain, 6×XRE sequence (a sequence of six mouse XRE sequences linked in tandem) derived from E. coli is preferably used. When a DNA sequence coding for a DNA-binding domain of E. coli repressor LexA is used as the DNA sequence coding for the DNA-binding domain, 8xLexA-46-P sequence (LexA-binding sequence repeated 8 times in series in an operator region (46 bases upstream from the transcription start point) of 35S promoter of cauliflower mosaic virus) is preferably used.

In a preferable embodiment, a vector according to the present invention may further comprise one ore more desirable genes. Examples of such a gene include drug resistance genes (e.g., NPTII (neomycinphosphotransferase II), genes coding for carnation THC2'GT, Arnt, flavonoid 3'-hydroxylase, flavonoid 3',5'-hydroxylase, and dihydroflavonol 4-reductase, and the like. These genes are preferably located under the control of an appropriate promoter (e.g., nopaline synthase promoter, Mac promoter).

A vector according to the present invention usually includes a DNA sequence corresponding to a nuclear localization signal sequence. The nuclear localization signal sequence localizes protein, which is a translation product of AhR gene according to the present invention, in a cell nucleus. Preferably, a nuclear localization signal sequence derived from SV40 is used, but there is no specific limitation on the nuclear localization signal sequence.

A vector according to the present invention usually includes an expression control region, an origin of replication, and the like such as, for example, an appropriate promoter and a terminator, depending on the type of host to which the vector is to be introduced. For example, in order to express a gene in a plant cell, (1) a promoter functional in the plant cell, (2) a gene, and (3) a DNA molecule (expression cassette) including a terminator functional in the plant cell in a functional manner, are produced and introduced into the plant cell. Such a DNA molecule may include a DNA sequence for enhancing the transcription, for example, an enhancer sequence, in addition to the promoter. Any promoter functional in a plant cell can be used, and examples of the useful promoter include 35S (Shcell, J. S., 1987, Science, 237:1176-1183), Nos (Schell, J. S., 1987, Science, 237:1176-1183), rbcS (Benefy, P. N. and N-H. Chua, 1989, Science, 244:174-181), PR1a (Ohshima, M. et al, 1990, Plant Cell, 2:95-106), ADH (Benefy, P. N. and N-H. Chua, 1989, Science, 244:174-181), patatin (Benefy, P. N. and N-H. Chua, 1989, Science, 244:174-181), Cab (Benefy, P. N. and N-H. Chua, 1989, Science, 244:174-181), PAL (Lian, X. et al., 1989, Proc. Natl. Acad. Sci. USA, 86:9284-9288) and the like.

### (Production of transgenic plant)

The above-described vector can be produced using a known restriction enzyme or the like in accordance with a known method. For example, when agrobacterium is used, a binary vector such as PBI121 or the like can be used. When a particle gun is used, an E. coli vector such as PUC 19 or the like can be used. Further, a plant transformed with a target gene can be obtained by subjecting plant cells transformed with such a vector to selection using a marker gene such as an antibiotic-resistant gene, and re-differentiating the selected plant cell with an appropriate plant hormone or other conditions.

The vector can be introduced into a plant cell by various methods well known to those skilled in the art. Examples of the useful method include indirect introduction methods using agrobacterium tumefaciens or agrobacterium rhizogenes (Heiei, Y. et al., Plant J., 6, 271-282, 1994, Takaiwa, F. et al., Plant Sci. 111, 39-49, 1995), and direct introduction methods represented by an electroporation method (Tada, Y. et al., Theor. Appl. Genet, 80, 475, 1990), a polyethyleneglycol method (Datta, S. K. et al., Plant Mol Biol., 20, 619-629, 1992), a particle gun method (Christou, P. et al., Plant J. 2, 275-281, 1992, Fromm, M. E., Bio/Technology, 8, 833-839, 1990), and the like. There is no specific limitation on the plant cell to which the gene is introduced according to the present invention, as long as the cell can be regenerated into a plant. Examples of the useful plant cell include suspension cultured cells, and cells forming callus, protoplast, piece of leaf or the like.

A transgenic plant cell can be regenerated into a plant. Accordingly, in another embodiment, the present invention also provides a transgenic plant transformed with a vector according to the present invention. Preferable plant species to be transformed with a vector according to the present invention include plant species in which anthocyanin has already been accumulated in the petal or anthocyanin is expected to be accumulated in the petal easily by gene introduction. Plant species with which a transformant is obtainable are more preferable. Examples of such plant species include petunia, torenia, verbena, tobacco, rose, chrysanthemum, carnation, snapdragon, cyclamen, orchid, lisianthus, freesia, gerbera, gladiolus, gypsophila, kalanchoe, lily, pelargonium, geranium, tulip and the like.

A plant, the flower color of which has been modified by introduction and expression of a vector according to the present invention, and also a plant obtained by a reproduction material thereof (e.g., seed, progeny, cut flower, tuberous root, tuber, fruit, cut spike, etc.), are encompassed in the technological scope of the present invention.

### (Method for detecting environmental chemical)

In still another embodiment, the present invention provides a method for detecting an environmental chemical (e.g., an AhR ligand) in soil using a transgenic plant transformed with a vector according to the present invention. The method includes the step of transplanting a transgenic plant transformed with a vector according to the present invention to the soil to be tested, cultivating the transgenic plant, and observing a change of flower color. When the flower color is changed (for example, when the flower color is paled and becomes closer to white), it is determined that an environmental chemical is detected. Representative environmental chemicals in soil include dioxins/dioxin-like compounds and/or polycyclic aromatic hydrocarbons, which are AhR ligands. Needless to say, a transgenic plant according to the present invention can be used to detect an environmental chemical contained in a culture medium, water, air or the like as well as in the soil.

Hereinafter, the present invention will be described in more detail by way of examples. The present invention is not limited to the following examples.

### [Examples]

In the following examples, the molecular biological techniques described in WO96/25500 or Molecular Cloning (Sambrook et al. (1989), Cold Spring Harbour Laboratory Press) were used unless otherwise specified.

In order to monitor dioxin or a dioxin-like compound through a change of flower color of a garden plant, the technology described in Japanese Laid-Open Patent Publication No. 2004-89068 regarding an AhR ligand-specific gene expression inductive factor and use of a heterologous gene inducible expression system based on the function of such a factor was applied. This technology utilizes two transcription structures. A first transcription structure includes a constitutive expression promoter and a DNA-binding domain - a nuclear localization signal sequence - a ligand-binding domain - a transcriptional activation domain under the control of the constitutive expression promoter. A second transcription structure includes a transcription-activating inductive promoter which acts when a ligand binds a protein synthesized as a result of transcription and translation from the first transcription structure, and a reporter gene below the inductive promoter (GUS, GFP, cytochrome P450 gene, or the like). In the examples, it was attempted to develop a plant which changes the flower color, by using a gene involved in the synthesis of anthocyanin (flower color gene) as a gene to be transcribed in the presence of dioxin or a dioxin-like compound, such a gene being a part of the second transcription structure. If such a plant is developed, it is expected that the flower color is changed only in the presence of dioxin or a dioxin-like compound. Such a plant is effective to monitor the state of environmental pollution in a simple manner and over a wide area.

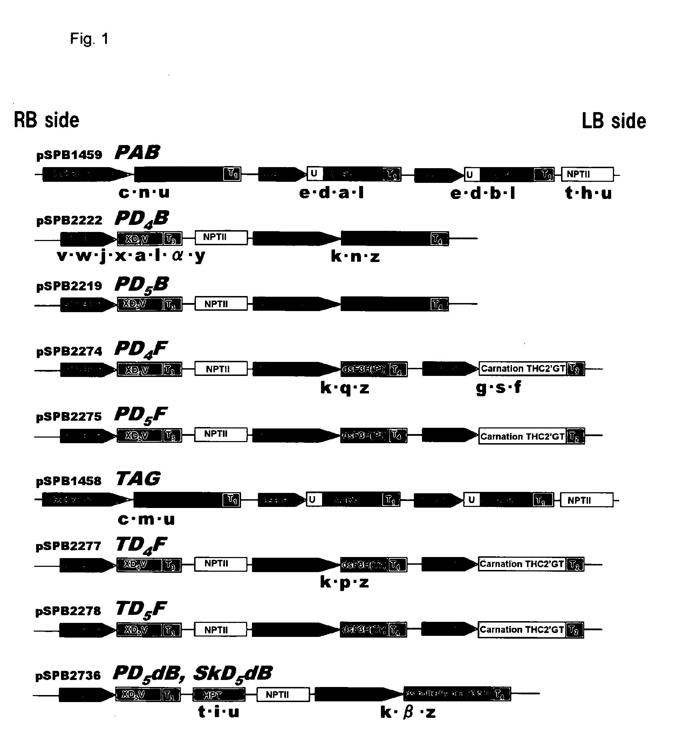

Three types of first transcription structures were produced. One was produced by replacing the transcriptional activation domain of mouse AhR with a transcriptional activation domain of human herpes simplex virus (VP16) (this transcription structure is referred to as "AhRV" (see Fig. 1)). The other two may be obtained by replacing the DNA-binding domain of AhRV with a DNA-binding domain of E. coli repressor LexA. As the other two first transcription structures, LexA-AhR83-494-VP16 and LexA-AhR83-593-VP16 described in Japanese Laid-Open Patent Publication No. 2004-89068 were used (these transcription structures are respectively referred to as "XD₄V" and "XD₅V" (see Fig. 1)). In XD₄V and XD₅V, an E. coli-derived sequence (LexA) is used as the DNA-binding domain. Therefore, these first transcription structures are not bound to 6xXRE sequence unlike AhRV, but to 8xLexA-46-P sequence (LexA-binding sequence repeated 8 times in series in an operator region (46 bases upstream from the transcription start point) of 35S promoter of cauliflower mosaic virus). XD₄V includes a sequence of amino acids No. 83 to No. 494 of mouse AhR as a domain binding to dioxin or a dioxin-like compound, and XD₅V includes a sequence of amino acids No. 83 to No. 593 of mouse AhR also as a domain binding to dioxin or a dioxin-like compound. Namely, for the AhR region, XD₅V uses an amino acid sequence which is 100 amino residue longer than XD₄V

First, AhRV, XD₄V and XD₅V were used as the three types of first transcription structures, and flavonoid 3'-5'-hydroxylase (F3'5'H) gene of butterfly pea was used as the second transcription structure. It was attempted to produce a garden plant which changes the flower color to purple in the presence of dioxin or a dioxin-like compound, so as to be useful to monitor dioxin or a dioxin-like compound. It was also attempted, using sGFP gene as a reporter gene of the second transcription structure, to produce a plant which can be used to check whether a target gene (second transcription structure) is expressed in response to dioxin.

### (Reference Example 1)

### Construction of construct for PAB, PD₄B, PD₅B and TAG

### Construction of construct for PAB

To binary vector pBinPlus (Trangenic Research 4, 288-290 (1995)) of pSPB748 (Plant Cell Physiol. 44, s122, 2003), El₂35S promoter sequence having an enhancer sequence repeated twice in a region upstream with respect to cauliflower mosaic virus 35S promoter (Plant Cell Physiol. 37, 49-59 (1996)), butterfly pea F3'5'H cDNA sequence (described in WO2004/020637) and nopaline synthase (nos) terminator sequence had been introduced. From pSPB748, a DNA fragment (about 2.0 kb) having butterfly pea F3'5'H cDNA and nos terminator linked to each other was recovered by BamHI digestion and EcoRI partial digestion and introduced into the BamHI/EcoRI site of pBluescriptII (sk-) (Stratagene). Thus, plasmid pB-Bn was obtained. To pBluescriptII (ks+) (Stratagene) of pBlueSXXREGUS, 6xXRE promoter sequence having mouse xenobiotic responsive element (XRE) repeated 6 times, GUS gene, and nos terminator had been introduced. From pBlueSXXREGUS, a gene cassette of GUS gene and nos terminator was excised by digestion with XbaI and KpnI. To this site, the DNA fragment (about 2.0 kb) having butterfly pea F3'5'H cDNA and nos terminator linked to each other, which was excised from pB-Bn by digestion with XbaI and KpnI, was inserted. Thus, pB-X6Bn was obtained. 5' non-translated (UTR) sequence of alfalfa mosaic virus were added to each of two sets of expression units formed of cauliflower mosaic virus 35S promoter and nos terminator on binary vector pBin19 (Nucl. Acids Res, 12, 8711-8721, 1984) to obtain AhRV and Arnt. To the SalI site of vector pSKAVAt having AhRV and Arnt introduced thereto in a forward direction, a gene cassette (2.2 kb) of 6xXRE promoter sequence, butterfly pea F3'5'H cDNA and nos terminator, which was excised by digestion with XhoI from pB-X6Bn, was introduced. Thus, pSPB1459 was constructed (see Fig. 1). pSPB1459 was introduced into agrobacterium tumefaciens strain Agl0 (BioTechnology 9, 963-967 (1991)), and petunia (brand name: PL, Skr4 xSw63 (see Nature, 366, 276-279)) was transformed by an agrobacterium method using a leaf disc. Introduction of plasmid into agrobacterium and transformation were performed in accordance with a known method (Plant J. 5, pp. 81-92, 1994). PL lacks flavonoid 3'-5'-hydroxylase and flavonoid 3'-hydroxylase, and therefore the flower color is white or pale pink. PL was used in this example, but the breed of petunia which can be used for the object of the present invention is not limited to PL. 38 lines of independent transgenic petunia PAB were obtained.

### Construction of construct for TAG

CaMV35S-sGFP(S65T)-NOS3'(Plant J, 18, 455-463, 1999) was digested with BamHI and EcoRI, and DNA (1.0 kb) having sGFP gene and nos terminator gene linked to each other was introduced into the BamHI/EcoRI site of pBluescriptII (sk-). Thus, plasmid pB-Gn was obtained. From pBlueSXXREGUS, a gene cassette (1.0 kb) of GUS gene and nos terminator was excised with XbaI and KpnI. To this site, a gene cassette (1.0 kb) of sGFP and nos terminator, which was excised from pB-Gn with XbaI and KpnI, was inserted. Thus, pB-X6Gn was constructed. To the SalI site of pSKAVAt, a gene cassette (1.2 kb) of 6×XRE promoter, sGFP and nos terminator, which was excised from pB-X6Gn by digestion with XhoI, was introduced. Thus, pSPB1458 was constructed (see Fig. 1).

pSPB1458 was introduced into agrobacterium tumefaciens starain Agl0, and transformed to torenia (breed: Summer Wave Blue (SBW; Suntoryflowers Co., Ltd.)) leaf disc by an agrobacterium method using a leaf disc. Transformation of torenia was performed in accordance with a known method (Suzuki et al. (2000) Mol. Breeding 6, pp. 239-246). The flower color of SWB is blue. SWB was used in this example, but the breed of torenia which can be used for the object of the present invention is not limited to SWB. 40 lines of independent transgenic torenia (referred to as "TAG") were obtained.

### Construction of construct for PD₄B and PD₅B

Binary vectors, that respectively has replacement of the GUS gene segment of the binary vector pGPΩD₄VGUS including LexA-AhR83-494-VP16 (XD₄V) and the GUS gene segment of the binary vector pGPΩD₅VGUS including LexA-AhR83-593-VP16 (XD₅V), which are described in Japanese Laid-Open Publication No. 2004-89068 as a system (XDV) in which gene expression is induced in an AhR ligand-specific manner, by butterfly pea F3'5'H cDNA, were constructed as follows. pGPΩD₄VGUS was digested with XhoI and SpeI to remove the GUS gene region, and to this site, an adaptor cassette of SpeIXhoI-F and SpeIXhoI-R oligonucleotides was inserted. Thus, pSPB2229 was constructed. Similarly, pSPB2221 was constructed from pGPΩD₅VGUS. Butterfly pea F3'5'H cDNA (about 1.7 kb) included in pBluescriptII (sk-) was digested with SpeI and XhoI to be recovered, and was linked to a DNA fragment obtained from pSPB2229 by digestion with SpeI and XhoI. Thus, pSPB2222 was constructed (see Fig. 1). Similarly, pSPB2219 was constructed from pSPB2221 and butterfly pea F3'5'H cDNA (see Fig. 1). As described above, pSPB2222 was introduced into agrobacterium tumefaciens strain Ag10, and the agrobacterium was used to transform the petunia PL lines. 42 lines of independent transgenic petunia (referred to as "PD₄B") were obtained. 38 lines of independent transgenic petunia having pSPB2219 introduced thereto (referred to as "PD₅B") were obtained.

The base sequences of SpeIXhoI-F and SpeIXhoI-R used above are as follows.
SpeIXhoI-F) 5'-tcgactagtccctcgag-3' (SEQ ID NO: 1)
SpeIXhoI-R) 5'-ctagctcgagggactag-3'(SEQ ID NO:2)

### (Reference Example 2)

### Induction experiment of PAB, PD₄B, PD₅B and TAG by dioxin or dioxin-like compound

An induction experiment of cultivating transgenic plants in the medium and soil containing dioxin or a dioxin-like compound to check whether or not a target gene (in this case, F3'5'H gene or GFP gene) was expressed to change the flower color was performed in two stages: an *in vitro* induction experiment performed in an agar medium using tissue-cultured seedling for the purpose of selecting high quality lines which can be induced (primary evaluation); and an induction experiment of cultivating in the soil and allowing the plants to flowering to check the change of flower color (secondary evaluation).

### Primary evaluation

For the primary evaluation, 20-methylcholanthrene (MC) was used as dioxin or a dioxin-like compound. MC is an analog of dioxin or a dioxin-like compound and is known as being bound to an AhR. PAB, PD₄B, PD₅B and TAG were transplanted to an MS agar medium (Physiol Plant, 15, 473-493, 1962) containing 5 µM 20-MC, and leaves and roots were recovered one week later. From each of the leaves and roots, total RNA was isolated using RNeasy Plant Mini Kit (QIAGEN). From 1 µg of total RNA thus obtained, cDNA was synthesized using Super Script^{™} First-Strand Synthesis System for RT-PCR (Invitrogen) in accordance with the protocol recommended by the manufacturer thereof. 1 µL of the obtained cDNA was used as a template. With PAB, PD₄B and PD₅B, in order to check the expression amount of butterfly pea F3'5'H, RT-PCR was performed using ChBHF-F and ChBHF-R as primers. With TAG, in order to check the expression amount of sGFP, RT-PCR was performed using EGFP-F1 and EGFP-R1 as primers. The reaction condition was that a cycle of 95°C for 1 minute, 55°C for 1 minute, and 72°C for 1 minute was repeated 30 times. The size of the products to be obtained was 0.4 kb for butterfly pea F3'5'H and 0.7 kb for sGFP. As a result of RT-PCR, an MC-specific increase of the expression amount of F3'5'H was observed in 8 lines out of 38 lines of PAB, 4 lines out of 42 lines of PD₄B, and 12 lines out of 38 lines of PD₅B. In TAG, an MC-specific increase of the expression amount of sGFP was observed in 5 lines out of 40 lines. From each of the 29 lines, 2 plants were obtained by vegetative proliferation, conditioned and transplanted to a flower pot. These were cultivated in greenhouse for about 1 month until flower buds were formed. Then, 1 plant of each line was used for the induction experiment using dioxin-contaminated soil (provided by National Institute for Agro-Environmental Sciences) (secondary evaluation).

The base sequences of the primers used are as follows.
ChBHF-F) 5'-agctcgtgcattcctcaaaacc-3' (SEQ ID NO: 3)
ChBHF-R) 5'-tcgattccgaaccctttgtctc-3' (SEQ ID NO:4)
EGFP-F1) 5'-atggtgagcaagggcagga-3' (SEQ ID NO:5)
EGFP-R1) 5'-ttacttgtacagctcgtccat-3' (SEQ ID NO:6)

### Secondary evaluation

The seedling of the 29 promising lines selected in the first evaluation were transplanted to dioxin-contaminated soil and monitored for the change of flower color and the expression of butterfly pea F3'5'H gene or the expression of sGFP gene for 6 weeks by RT-PCR. The dioxin concentration of the dioxin-contaminated soil used was 360 pg-TEQ/g, which is lower than the national environmental standard of 1000 p-TEQ/g. The flowers and leaves were recovered, the RNA was recovered as described above, and RT-PCR was performed. Dioxin-specific expression of butterfly pea F3'5'H gene was confirmed in the flower buds of 4 lines out of 8 lines of PAB and 5 lines out of 12 lines of PD₅B. In the flower buds of PD₄B, expression of F3'5'H gene was not confirmed in any of the 4 lines. In the flower buds of total 9 lines of PAB and PD₅B in which F3'5'H gene was expressed, the flower color was pink like the flower color of the PL line into which the gene had been introduced. Thus, no change of flower color was confirmed. With TAG, only leaves were sampled for performing RT-PCR. In 4 lines out of 5 lines, dioxin-specific expression of sGFP was confirmed.

From the above results, it was confirmed that both in petunia and torenia, in the case where dioxin or a dioxin-like compound is present in the soil, butterfly pea F3'5'H gene or sGFP gene as the second transcription structure is induced in the flowers or leaves. However, the flower color did not change probably because the expression amount of the genes was insufficient.

### (Example 1)

### Construction of construct for PD₄F, PD₅F, TD₄F and TD₅F

It is considered that in the attempts to induce the expression of butterfly pea F3'5'H gene in petunia PL with dioxin or a dioxin-like compound as performed in Reference Examples 1 and 2, the flower color did not change because the induction level was too low. Now, it was attempted to specifically suppress the endogenous gene involved in the synthesis of anthocyanin by dioxin or a dioxin-like compound and thus to suppress the synthesis of anthocyanin and thus change the flower color.

In this example, as the gene in the second transcription structure involved in the synthesis of anthocyanin, flavanone 3-hydroxylase (F3H) was used, and as the gene expression suppressing method, the RNAi method was used. F3H is an enzyme catalyzing the reaction of converting naringenin into dihydrokaempferol. By suppressing the expression of F3H gene, the biosynthesis of flavonoid downstream of dihydrokaempferol is inhibited. Since anthocyanin is not accumulated due to the suppression on the F3H gene expression, the flower color exhibited by the accumulation of anthocyanin, which is originally a colored pigment, is changed to a color closer to white.

Two types of first transcription structures, i.e., XD₄V and XD₅V were used. As the plants, petunia and torenia were used.

### Construction of construct for PD₄F

The construct for petunia dsF3H was produced by the method described in WO2004/018682, example 8, for constructing a petunia F3H cDNA double strand construct.

To the site at which pSPB560, having Mac promoter and mas terminator on a vector derived from pUC, was digested with SpeI and SacI, a cDNA gene fragment PhF3H-1 (SacI/ClaI fragment: 0.7 kb) and PhF3H-1' (SpeI/ClaI fragment: 0.9 kb) of petunia F3H were introduced by three-fragment DNA ligation. Thus, pSPB1497 was constructed. A gene cassette of petunia dsF3H was excised from pSPB 1497 by SpeI digestion, and inserted into the SpeI site of pSPB2229 mentioned above. Thus, pSPB2249 was constructed. From pSPB1500 described in WO2004/018682, example 6, a gene cassette (3.6 kb) formed of Mac promoter, chalcone 2' glucosyltransferase (T170) gene, and mas terminator was excised with AscI, and blunted using DNA Blunting Kit (Takara) (hereinafter, all the blunting operations were performed using DNA Blunting Kit). Thus, an MT170m fragment was obtained. pSPB2249 was digested with SacII and end-blunted. To this site, MT170m was inserted into construct pSPB2274 (see Fig. 1). In Fig. 1, "carnation THC2'GT" is an enzyme for transglycosylating position 2' of chalcone, which is a yellow pigment. Carnation THC2'GT prevents chalcone from being changed to naringenin, which is a colorless pigment, by chalcone isomerase or the automatic ring closure of chalcone itself, and thus stabilizes chalcone. For these reasons, "carnation THC2'GT" is inserted for the purpose of changing the flower color to yellow. However, because the yellow pigment of chalcone was pale and the amount of accumulated THC glycoside was small, the flower color was not changed to yellow as expected.

As described above, pSPB2274 was introduced into agrobacterium tumefaciens strain Ag10 to transform red petunia. This breed lacks flavonoid 3',5'-hydroxylase gene, and so the flower color thereof is red. Petunia used for the object of the present invention is not limited to red breeds, and may be of any color as long as it is not colorless. 16 lines of independent transgenic petunia (referred to as "PD₄F") were obtained.

### Construction of construct for PD₅F

A gene cassette of petunia dsF3H was excised from pSPB1497 with SpeI digestion and inserted into the SpeI site of pSPB2221 mentioned above to construct pSPB2250. An MT170m fragment was obtained from pSPB1500 and inserted into a site at which pSPB2250 was digested with SacII and blunted. Thus, pSPB2275 was constructed (see Fig. 1). As described above, pSPB2274 was introduced into agrobacterium tumefaciens strain Ag10 to transform petunia "Baccara Red". 15 lines of independent transgenic petunia (referred to as "PD₅F") were obtained.

### Construction of construct for TD₄F

cDNA coding for F3H of torenia was obtained as follows using F3H cDNA obtained from perilla (Zhizhong Gong et al. (1997) Plant Mol Biol. 35, pp. 915-927) as a probe. The probe was labeled by PCR using a non-radioisotope DIG-nuclear acid detection system (Roche Diagnostics) in accordance with the conditions recommended by the manufacturer thereof As the template, plasmid containing 1 ng of cDNA was used, and 100 ng of oligonucleotide specific to each of the above-mentioned genes was used as the primer. PCR was performed with a cycle of 95°C for 1 minute, 55°C for 1 minute, and 72°C for 2 minutes, which was repeated 25 times. Using a PCR sub product of perilla F3H gene as a probe for hybridization, about 200,000 phages of torenia cDNA library (Suzuki et al. (2000) Mol. Breeding 6, pp. 239-246) were screened. The hybridization was carried out in 5 × SSC containing 30% formaldehyde at 37°C overnight, and the filter was washed using 5 × SSC, 1% SDS at 55°C for 30 minutes. The cDNA sequence was determined by a primer walking method using a synthetic oligonucleotide primer. The base sequence of the amino acid coded region of the cDNAs is shown in sequence ID No. 7 in the Sequence Listing. The obtained torenia F3H cDNA was contained in vector pBluescriptII (sk-) and is referred to as "plasmid pSPB266". Using this as a template, PCR was performed using M13 reverse primer (SEQ ID NO:8) derived from the vector sequence and primer ThF3H-SalI-1 (SEQ ID NO:9), in which a SAlI recognition site was inserted into torenia F3H cDNA sequence by base substitution. The obtained fragment of about 0.9 kb was cloned to pCR2.1-TOPO (Invitrogen) to confirm the base sequence. Similarly, a DNA fragment of about 0.75 kb was prepared using primer ThF3H-SalI-2 (SEQ ID NO:10), in which a SAII recognition site was inserted into torenia F3H cDNA sequence by base substitution, and M13RV primer. The obtained DNA fragment was cloned to pCR2.1-TOPO to confirm the base sequence.

pBE2113-GUS (Plant Cell Physiol. 37, 45 (1996)) was digested with SnaBI, and a part corresponding to the 5' side of GUS gene was removed from omega sequence which was downstream of the promoter. To this part, a BamHI linker (Takara) was inserted. The obtained plasmid was referred to as "pUE6". pUE6 was digested with HindIII and EcoRI, and a fragment including the GUS expression cassette was recovered. This fragment was inserted into the HindIII and EcoRI site of pUCAA, and the obtained plasmid was referred to as "pSPB541". pSPB541 was digested with BamHI and SacI, and the GUS gene part was removed. To this part, 0.9 kb of fragment excised from pCR2.1-TOPO by being digested with BamHI and SalI and 0.7 kb of fragment excised from pCR2.1-TOPO by being digested with SacI and SalI were inserted, such that the SalI sites of both fragments would be linked to each other. Plasmid pSFL313 obtained in this manner was constructed to express double strand RNA derived from the torenia F3H gene sequence under the control of El₂35S promoter when introduced into a plant and to suppress the expression of torenia F3H gene by the RNAi method.

The base sequences of the primers used are as follows.
M13RV) 5'-caggaaacagctatgac-3' (SEQ ID NO:8)
ThF3H-SalI-1) 5'-ttctctgtcgacgcccattgcc-3' (SEQ ID NO:9)
ThF3H-SalI-2) 5'-cgccgtgtcgactcgcttgaag-3' (SEQ ID NO: 10)

A gene cassette (1.6 kb) of torenia dsF3H was excised from pSFL313 with BamHI and introduced into the BamHI site of pBluescriptII (sk-) to construct pSPB2251. A gene cassette of torenia dsF3H was excised from pSPB2251 with SpeI and EcoRV. pGPΩD₄VGUS mentioned above was digested with XhoI and blunted, and inserted into the site at which digestion with SpeI was performed. Thus, pSPB2260 was constructed. pSPB2260 was digested with SacII and blunted, and to this site, the MT170m fragment mentioned above (3.6 kb) was introduced. Thus, pSPB2277 was constructed (see Fig. 1). As described above, pSPB2277 was introduced into agrobacterium tumefaciens strain Ag10 to transform torenia SWB. 40 lines of independent transgenic torenia (referred to as "TD₄F") were obtained.

### Construction of construct for TD₅F

A gene cassette (1.6 kb) of torenia dsF3H excised from pSPB2251 with SpeI and EcoRV was inserted into a site, at which pGPΩD₅VGUS was digested with XhoI, blunted, and then digested with SpeI. Thus, pSPB2261 was obtained. pSPB2261 was digested with SacII and blunted, and to this site, the MT170m fragment mentioned above (3.6 kb) was introduced. Thus, pSPB2278 was constructed (see Fig. 1). pSPB2278 was introduced into agrobacterium tumefaciens strain Ag10 to transform torenia SWB. 40 lines of independent transgenic torenia (referred to as "TD₅F") were obtained.

### (Example 2)

### Induction experiment of PD₄F, PD₅F, TD₄F and TD₅F by dioxin or dioxin-like compound

Unlike PAB, PD₄B, PD₅B and TAG having extrinsic genes (butterfly pea F3'5'H, sGFP) introduced thereto, in the case of PD₄f, PD₅F, TD₄F and TD₅F having a construct for suppressing endogenous gene (F3H) introduced thereto, the expression of F3H is observed only in the petal. Therefore, the influence of dioxins/dioxin-like compounds cannot be observed unless the flower buds of the plant are sampled. For this reason, the primary evaluation described in Reference Example 2 was not performed on any of PD₄F, PD₅F, TD₄F and TD₅F, and the induction experiment of only the secondary evaluation was performed. The obtained transformants were all transferred to a flower pot, and then transplanted to soil contaminated with dioxin or a dioxin-like compound and cultivated there.

### Secondary evaluation

16 lines of PD₄F, 15 lines of PD₅F, 48 lines of TD₄F and 40 lines of TD₅F were transplanted to soil contaminated with dioxin or a dioxin-like compound like in the secondary evaluation in Reference Example 2 (concentration of dioxin or a dioxin-like compound: 360 pg-TEQ/g), and the change of the flower color was observed for 4 weeks.

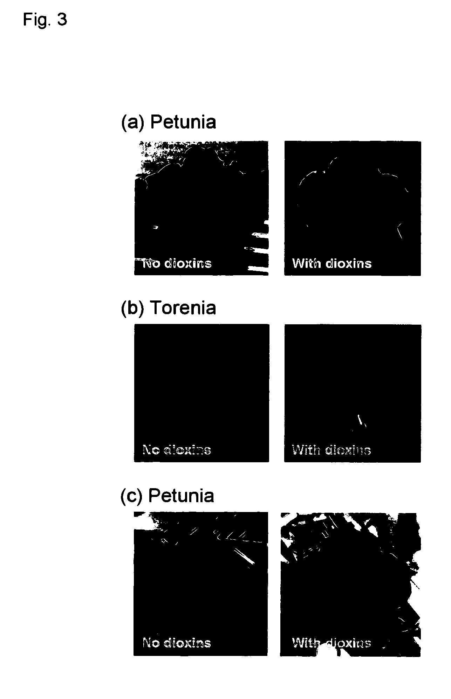

As shown in Fig. 3(a) and (b), it was observed that the flower color changed to white in all the lines. Regarding all the lines, the color change was considered to have occurred as a result of the expression of dsF3H specific to dioxin or a dioxin-like compound. As described above, transformants of petunia and torenia exhibiting the change of the flower color in the case where dioxin or a dioxin-like compound is present in the soil were obtained. It was confirmed that the contamination of soil with dioxin or a dioxin-like compound can be monitored by checking the flower color of the PD₄F and PD₅F lines of petunia and TD₄F and TD₅F lines of torenia.

### (Example 3)

### Production of transgenic petunia P3B and verbena sk3B

pSPB748 was introduced into agrobacterium tumefaciens strain Ag10 by the above-described method, and the resultant agrobacterium was used to transform petunia PL. As a result, 27 lines of independent transgenic petunia P3B were obtained. The flower color of petunia PL is pale pink, but the flower color of the obtained lines was reddish purple. Similarly, the same agrobacterium was used to transform verbena (breed: Hanatemari Sakura (Suntoryflowers Co., Ltd.)). The transformation was performed by a known method (Plant Cell Rep. 21, 459-466, 2003). As a result of the transformation, 6 lines of independent transgenic verbena Sk3B were obtained. The flower color of Hanatemari Sakura is pink, but the flower color of the obtained lines was purple.

### Construction of construct for PD₅dB and SkD₅dB

A binary vector pSPB2219 (construct for PD₅B) including LexA-AhR83-593-VP16 (XD₅V), in which the butterfly pea F3'5'H gene part is replaced with butterfly pea dsF3'5'H cDNA and to which an HPT gene cassette formed of 35S promoter - hygromycin-resistant (HPT: hygromycin phosphotransferase) gene - nos terminator is incorporated, was constructed as follows. pSPB2219 was digested with SacII and end-blunted. From a binary vector pLAN101MHYG (Plant Cell Physiol. 41, 1397-1406, 2000) including the HPT gene cassette, the HPT gene cassette was excised by digestion with HindIII and end-blunted. The obtained HPT gene cassette was inserted into the site at which digestion with SacII was performed. Thus, pSPB2295 was constructed. Next, to the site at which the pBluescriptII (sk-) vector was digested with SpeI and BamHI, a cDNA gene fragment BHF-1 of butterfly pea dsF3'5'H (fragment (0.6 kb) digested with SpeI and HincII and end-blunted) and a cDNA gene fragment BHF-1' butterfly pea F3'S'H (fragment (0.9 kb) digested with BamHI/FokI and end-blunted) both obtained from pSPB748 were introduced with 3-fragment DNA ligation. Thus, pSPB2727 having the butterfly pea dsF3'5'H gene cassette (1.5 kb) was constructed. pSPB2295 mentioned above was digested with XhoI and SpeI to remove the butterfly pea F3'5'H gene region. To this site, the butterfly pea dsF3'5'H gene cassette, which was excised from pSPB2727 by digestion with XhoI and SpeI, was inserted. Thus, pSPB2736 was constructed (see Fig. 1).

### Production of transgenic petunia PD₅dB and verbena SkD₅dB

P3B-3 line, which exhibited the highest level of flower color change among P3B obtained in the above-described transformation, was used as a parent line to perform transformation using agrobacterium having pSPB2736 introduced thereto. Thus, 13 lines of independent transgenic petunia PD₅dB were obtained. Similarly, Sk3B-5 line, which exhibited the highest level of flower color change among Sk3B, was used as a parent line to perform transformation. For the transformation, a known method (Plant Cell Rep. 21, 459-466, 2003) was partially modified and selection with hygromycin was performed. As a result of the transformation, 14 lines of independent transgenic verbena SkD₅dB were obtained.

### Induction experiment of PD₅dB and SkD₅dB by dioxin-contaminated soil

The transgenic plants were cultivated in soil containing dioxin or a dioxin-like compound, and an induction experiment was performed with the soil cultivation in order to check whether the expression of the butterfly pea dsF3'5'H gene was suppressed to change the flower color.

From each of the PD₅dB and SkD₅dB lines, 2 plants were obtained by vegetative proliferation, conditioned and transplanted to a flower pot. These were cultivated in greenhouse for about 1 to 2 months until flower buds were formed. Then, 1 plant of each line was transplanted to dioxin-contaminated soil (provided by National Institute for Agro-Environmental Sciences). PD₅dB was cultivated for 10 weeks, SkD₅dB was cultivated for 14 weeks, and the change of flower color was observed. The concentration of the dioxin or a dioxin-like compound in the dioxin-contaminated soil used was as described above.

As shown in Fig. 3(c), with PD₅dB, the flower color of some of the lines was changed to pink. With SkD₅dB, because the ratio of flower color change was low in all the lines, a gene expression analysis by real-time PCR was performed. The lines were transplanted to dioxin-contaminated soil, and leaves were sampled from verbena in the 14th week. The RNA was recovered by the above-described method and cDNA was synthesized. In order to check the expression amount of butterfly pea dsF3'5'H, real-time PCR was carried out, using 1 µl of the obtained cDNA as a template, BHF-F and BHF-R as a primer, and BHF-T as a probe, and using TaqMan Universal PCR Master Mix (Applied Biosystems) in accordance with the protocol recommended by the manufacturer thereof. In order to check the expression amount in non-contaminated soil as a comparative example, the remaining 1 plant of each line was cultivated in greenhouse without being transplanted to the contaminated soil, and the real-time PCR was performed in substantially the same manner. The results are shown in Table 1.

**(Table 1)**

| SkD₅dB | 14th week |
|---|---|
| 1 | 0.07 |
| 2 | 0.29 |
| 3 | 0.47 |
| 4 | 0.14 |
| 5 | 0.50 |
| 6 | 0.79 |

The numerals in the table represent the ratio of the expression amount of butterfly pea F3'5'H gene in the leaves when verbena transformed with a vector according to the present invention was cultivated in the absence and in the presence of dioxin or a dioxin-like compound, i.e., the expression amount in the presence of dioxin or a dioxin-like compound/the expression amount in the absence of dioxin or a dioxin-like compound.

As is clear from Table 1, in the verbena transformed with the vector according to the present invention, the expression of the butterfly pea F3'5'H gene was suppressed by dioxin or a dioxin-like compound.

The base sequences of the primers and the probe used are as follows.
BHF-F) 5'-GCTCATCATACCAACGAGTCTCA-3'(SEQ ID NO:11)
BHF-R) 5'-GATGATGTATCTGTGCCTGCAGT-3'(SEQ ID NO: 12)
BHF-T) 5'-FAM-AACTGTCGCTCACCAACATCAAAGCACTC-TAMRA-3' (SEQ ID NO: 13)

### INDUSTRIAL APPLICABILITY

A vector according to the present invention can be used for transforming a plant cell, and a plant transformed with a vector according to the present invention can change the flower color thereof in response to an AhR ligand. Accordingly, such a transgenic plant is useful for a simple, quick, highly-sensitive and low-cost detection method for detecting the presence of an environmental chemical such as dioxins/dioxin-like compounds, while allowing an on-site measurement and not imposing much load on the environment.

## Claims

1. A plant transformation vector for producing a plant for detecting an environmental chemical by a change of flower color, the plant transformation vector comprising an AhR gene expressibly incorporated therein, an AhR ligand stimulus-inducible promoter, and an expression suppressive factor for suppressing expression of a gene involved in the synthesis of a desired flower pigment, the factor being expressibly incorporated therein by the promoter.

2. The plant transformation vector according to claim 1, comprising genes coding for a DNA-binding domain, an AhR ligand-binding domain and a transcriptional activation domain, also comprising a nucleic acid coding for a double strand RNA having an RNA interference effect on the gene involved in the synthesis of the flower pigment; wherein the nucleic acid is under the control of the AhR ligand stimulus-inducible promoter, and the DNA-binding domain binds the AhR ligand stimulus-inducible promoter in response to an AhR ligand.

3. The plant transformation vector according to claim 1, wherein the gene involved in the synthesis of the flower pigment is a gene involved in the biosynthesis of anthocyanin.

4. The plant transformation vector according to claim 3, wherein the gene involved in the biosynthesis of anthocyanin is a gene coding for flavanone 3-hydroxylase (F3H) or flavonoid 3',5'-hydroxylase (F3',5'H).

5. The plant transformation vector according to claim 1, wherein an AhR ligand is any of dioxins/dioxin-like compounds, and/or polycyclic aromatic hydrocarbons, or an analog thereof.

6. The plant transformation vector according to claim 1, comprising XD₄V or XD₅V

7. The plant transformation vector according to claim 1, wherein the AhR ligand stimulus-inducible promoter includes a LexA promoter sequence.

8. The plant transformation vector according to claim 1, further comprising at least one desired gene.

9. The plant transformation vector according to claim 8, wherein the desired gene is a drug resistance gene.

10. The plant transformation vector according to claim 8, wherein the desired gene is carnation THC2'GT gene.

11. The plant transformation vector according to claim 1, wherein the plant is petunia, torenia or verbena.

12. A transgenic plant for changing the flower color thereof in response to an AhR ligand, the transgenic plant being transformed with the vector according to claim 1.

13. The transgenic plant according to claim 12, which is petunia, torenia or verbena.

14. A reproduction material of the transgenic plant according to claim 12.

15. A method for detecting an AhR ligand in soil using a transgenic plant, the method comprising the step of transplanting the transgenic plant according to claim 12 to soil to be tested, cultivating the transgenic plant, and observing a change of flower color.

16. The method according to claim 15, wherein the AhR ligand is any of dioxins/dioxin-like compounds, and/or polycyclic aromatic hydrocarbons, or an analog thereof.


<110>  Suntory Limited

<120>  Transgenic plant for detecting environmental chemicals

<130>  G07-0062

<150>  JP2005-036338
<151>  2005-02-14

<160>  13

<170>  PatentIn version 3.3

<210>  1
<211>  17
<212>  DNA
<213>  Artificial

<220>
<223>  SpeIXhoI-F

<400>  1
tcgactagtc cctcgag                                                    17


<210>  2
<211>  17
<212>  DNA
<213>  Artificial

<220>
<223>  SpeIXhoI-R

<400>  2
ctagctcgag ggactag                                                    17


<210>  3
<211>  22
<212>  DNA
<213>  Artificial

<220>
<223>  ChBHF-F

<400>  3
agctcgtgca ttcctcaaaa cc                                              22

<210>  4
<211>  22
<212>  DNA
<213>  Artificial

<220>
<223>  ChBHF-R

<400>  4
tcgattccga accctttgtc tc                                              22

<210>  5
<211>  19
<212>  DNA
<213>  Artificial

<220>
<223>  EGFP-F1

<400>  5
atggtgagca agggcagga                                                  19

<210>  6
<211>  21
<212>  DNA
<213>  Artificial

<220>
<223>  EGFP-R1

<400>  6
ttacttgtac agctcgtcca t                                               21

<210>  7
<211>  1101
<212>  DNA
<213>  Torenia fournieri

<400>  7
atggcacgag caggaccact aaccctaact tcgctagcgc tcgagaaatc gctgcatgaa      60

aagtttataa gggacgaaga cgagaggcct aacttagcat acgatcaatt tagcagtcag 120

attccattga tctctctctc tgggatcgac gatgaatgta ataagaggaa agagctgtgc 180

aagagaatag cgcaggcatg cgaagattgg ggtatttttc aagtgatcga tcatgggatc 240

gatttgaaac tcgtcaacga tatgactcgt ttggctcgtg agttcttcga tttgcccgac 300

gaagagaagc tgaggttcga tatgtctggt gggagaaaag gaggtttcat tgtttcgagc 360

caccttcagg gcgaggtggt ccaagactgg cgcgagatcg tgacctactt cacataccct 420

atcaaaggcc gtgactattc cctgtggccc gacaagcccg aggcatggcg ggccgtgaca 480

gagacctaca gctcgcagct aatgtgcctg ggctgcaaat tgctaggaat cctatccgag 540

gcaatgggcc tcgaaagaga agcgctgacc aaggcctgtc tgaacatgga ccaaaaagtt 600

gtggtcaact tttacccaaa atgccctcag cccaatttga cattgggcct gaagaggcac 660

tcggacccag gtttgatcac tctgctgttt caggataacg ttggcgggct tcaagcgact 720

cgagacggcg ggaagtcgtg gatcacggtc cagcccgttg agggtgcatt cgtggtcaat 780

cttggtgatt ttgctcatta cttgagcaat ggaaggttca agaacgcgga tcatcgagcg 840

gtggtgaatt caaacacgaa tagaatgtcg atcgcgacgt ttcaaaaccc atcgccagag 900

gctatcgtgt accctctcaa gatcggagac gacgggaagc ccattataga aaagcccatc 960

acttatggag aaatgtacaa gaggaagatg gctaaagaca ttgaacttgc caagctcaag 1020

aagctagcca aggaacaaaa gttgcaagaa gaagttgtta ataatgttga agatcatcat 1080

cttaacaatg ggaaaactaa a 1101

<210> 8
<211> 17
<212> DNA
<213> Artificial

<220>
<223> M13RV

<400> 8

caggaaacag ctatgac 17

<210> 9
<211> 22
<212> DNA
<213> Artificial

<220>
<223> ThF3H-SalI-1

<400> 9
ttctctgtcg acgcccattg cc 22

<210> 10
<211> 22
<212> DNA
<213> Artificial

<220>
<223> ThF3H-SalI-2

<400> 10
cgccgtgtcg actcgcttga ag 22

<210> 11
<211> 23
<212> DNA
<213> Artificial

<220>
<223> BHF-F

<400> 11
gctcatcata ccaacgagtc tca 23

<210> 12
<211> 23
<212> DNA
<213> Artificial

<220>
<223> BHF-R

<400>  12
gatgatgtat ctgtgcctgc agt                                              23

<210>  13
<211>  29
<212>  DNA
<213>  Artificial

<220>
<223>  BHF-T; additionally having reporter dye FAM at the 5' end and
       quencher dye TAMRA at the 3' end

<400>  13
aactgtcgct caccaacatc aaagcactc                                        29